# EUROPÄISCHE PATENTSCHRIFT

(11) **EP 1 958 659 B1**
(45) Veröffentlichungstag und Bekanntmachung des Hinweises auf die Patenterteilung: **26.08.2009**
(21) Anmeldenummer: 07013306.1
(22) Anmeldetag: 06.07.2007
(51) Int. Cl.: A61M 37/00, A01K 11/00

(54) **Antriebsmodul für eine Vorrichtung zum lokalen Aufstechen einer menschlichen oder einer tierischen Haut und Handgerät**
Drive module for a device for local puncturing of human or animal skin and hand-held device
Module d'entraînement pour un dispositif de perçage local d'une peau humaine ou animale et appareil manuel

(30) Priorität: 14.02.2007 EP 07003119
(43) Veröffentlichungstag der Anmeldung: 20.08.2008
(73) Patentinhaber: MT Derm GmbH, 14167 Berlin (DE)
(72) Erfinder: Lisec, Walter, 13053 Berlin (DE); Adler, Frank, 13053 Berlin (DE)
(74) Vertreter: Bittner, Thomas L.

(56) Entgegenhaltungen:
- EP-A- 1 495 782
- DE-U1- 29 919 199
- US-A1- 2004 143 275

## Beschreibung

Die Erfindung betrifft ein Antriebsmodul für eine Vorrichtung zum lokalen Aufstechen einer menschlichen oder einer tierischen Haut sowie ein Handgerät.

### Hintergrund der Erfindung

Vorrichtungen zum lokalen Aufstechen einer menschlichen oder einer tierischen Haut werden in der Regel als Handgeräte ausgeführt. Von Bedienpersonal können derartige Handgeräte zum Aufbringen einer Farbe für eine Tätowierung und / oder permanentes Make-up im Bereich der Hautoberfläche verwendet werden. Aber auch das Einbringen kosmetischer oder medizinischer Wirkstoffe über die Haut ist mit solchen Geräten möglich. Darüber hinaus können solche Geräte verwendet werden, ohne dass irgendeine Substanz eingebracht wird.

Ein Handgerät zum lokalen Aufstechen einer Haut ist beispielsweise aus der Druckschrift DE 299 19 199 U bekannt. Das bekannte Handgerät umfasst ein Griffstück, einen Nadelantrieb, eine Nadel und eine Nadeldüse, wobei mindestens zwei lösbar miteinander verbundene Module vorgesehen sind und das eine der beiden Module als wieder verwendbares Grundmodul mit integriertem Nadelantrieb ausgebildet ist. Das andere der beiden Module ist ein sterilisiertes Einwegmodul, in das alle durch die Köperflüssigkeiten eines Kunden infizierbaren Bestandteile des Handgeräts integriert sind. Auf diese Weise wird das Handgerät in Form von zwei Modulen zur Verfügung gestellt, von denen eines, nämlich das Einwegmodul, nach der Benutzung ausgetauscht werden kann, während das andere Modul, welches den Nadelantrieb umfasst, wieder verwendet wird. Mit Hilfe des Einwegmoduls werden die hygienischen Bedingungen beim Aufbringen einer Tätowierung und/oder von permanentem Make-up verbessert, da alle Teile ausgetauscht werden, die potentiell durch die bei der Behandlung austretende Körperflüssigkeit des Kunden infiziert werden können. Es wird so vermieden, dass das gesamte Handgerät ausgetauscht werden muss.

Aus dem Dokument EP 1 495 782 ist ein Antriebsmodul für eine Vorrichtung zum lokalen Aufstechen einer menschlichen oder einer tierischen Haut bekannt, bei dem eine Antriebseinrichtung, mit der eine Antriebsbewegung erzeugbar ist, und ein an die Antriebseinrichtung gekoppelter Wandlungsmechanismus vorgesehen sind, mit dem die Antriebsdrehbewegung in einer auf eine die Haut lokal aufstechende Nadeleinrichtung einkoppelbare Vorwärts- / Rückwärtsbewegung umgewandelt wird, so dass eine repetierende Bewegung der Nadeleinrichtung ermöglicht ist. Der Wandlungsmechanismus umfasst ein Funktionsbauteil, welches bei der Bewegungswandlung einen Taumel- oder Kippbewegung ausführt, wodurch eine Antriebskraft zum Bewegen einer die Haut lokal aufstechende Nadeleinrichtung in Vorwärts- und Rückwärtsrichtung zur Verfügung gestellt wird. Das Funktionsbauteil ist in einer Ausführung mittels eines Kugellagers freilaufend gelagert. Ein nicht beabsichtigtes Verdrehen des Funktionsbauteils, welches durch die Antriebsdrehbewegung hervorgerufen werden kann, wird bei der bekannten Vorrichtung dadurch verhindert, dass das mittels Kugellager montierte Funktionsbauteil oder ein hieran gebildeter Vorsprung in eine Ausnehmung eingreifen. Es hat sich gezeigt, dass diese Art der Sicherung des Funktionsbauteils beim Betrieb der Vorrichtung zur einer nicht unerheblichen Geräuschbelastung führt, die insbesondere durch das Hin- und Herbewegen des Vorsprungs in der Ausnehmung verursacht wird.

### Zusammenfassung der Erfindung

Aufgabe der Erfindung ist es, ein Antriebsmodul für eine Vorrichtung zum lokalen Aufstechen einer menschlichen oder einer tierischen Haut sowie ein Handgerät anzugeben, bei denen die Geräuschbelastung für den Benutzer beim Betrieb minimiert ist. Weiterhin sollen Vibrationen beim Betrieb der Vorrichtung vermindert werden.

Diese Aufgabe wird erfindungsgemäß durch ein Antriebsmodul nach dem unabhängigen Anspruch 1 sowie ein Handgerät nach dem unabhängigen Anspruch 12 gelöst.

Die Erfindung umfasst den Gedanken, bei einem Antriebsmodul für eine Vorrichtung zum lokalen Aufstechen einer menschlichen oder einer tierischen Haut eine Antriebseinrichtung, die konfiguriert ist, eine Antriebsdrehbewegung zu erzeugen, und einen an die Antriebseinrichtung gekoppelten Wandlungsmechanismus vorzusehen, der konfiguriert ist, die Antriebsbewegung in eine auf eine die Haut lokal aufstechende Nadeleinrichtung einkoppelbare Vorwärts- / Rückwärtsbewegung zu wandeln, wobei der Antriebsmechanismus ein freilaufendes Funktionsbauteil umfasst, welches an eine eine Taumel- oder Kippbewegung des Funktionsbauteils zulassende, magnetische Verdrehsicherung gekoppelt ist, mit der eine einem Verdrehen des Funktionsbauteils entgegenwirkende, magnetische Rückhaltekraft zur Verfügung gestellt ist. Mit Hilfe der magnetischen Verdrehsicherung ist eine individuell an verschiedene Ausgestaltungen des Antriebsmoduls anpassbare Möglichkeit geschaffen, eine ausreichende Rückhaltekraft zur Verfügung zu stellen, die ein unbeabsichtigtes Verdrehen des Funktionsbauteils zuverlässig verhindert. Mit der Taumel- oder Kippbewegung des Funktionsbauteils wird die Antriebsdrehbewegung einer Antriebswelle an der Antriebseinrichtung in eine lineare, repetierende Bewegung umgewandelt. Um eine möglichst gute Reproduzierbarkeit der Vorwärts- / Rückwärtsbewegung zu erhalten, soll ein Verdrehen des Funktionsbauteils nahezu oder vollständig ausgeschlossen sein. Mit Hilfe der magnetischen Verdrehsicherung wird die als solche bekannte mechanische Verdrehsicherung, welche in bekannten Ausführungen zu einer erheblichen Geräuschbelastung führt, ergänzt oder sogar vollständig ersetzt.

Mittels geeigneter Wahl der magnetische Rückhaltekraft kann die Verdrehsicherung kontaktfrei oder unter Einbeziehung einer Kontaktausbildung zwischen Elementen der Verdrehsicherung ausgeführt werden. Bei ausreichender Stärke der magnetischen Rückholkraft können die Elemente der Verdrehsicherung auch im Betrieb beabstandet gehalten werden.

Eine bevorzugte Weiterbildung der Erfindung sieht vor, dass die magnetische Verdrehsicherung als berührungslose Verdrehsicherung gebildet ist, die konfiguriert ist, die magnetische Rückhaltekraft berührungslos auf das Funktionsbauteil einzukoppeln. Mittels einer berührungslosen Kopplung werden durch die magnetische Verdrehsicherung eventuell entstehende Geräusche im wesentlichen vollständig ausgeschlossen.

Bei einer zweckmäßigen Ausgestaltung der Erfindung kann vorgesehen sein, dass die magnetische Verdrehsicherung mit einem oder mehreren Magnetelementen gebildet ist, die wahlweise an dem Funktionsbauteil und / oder an einem feststehenden Bauteil angeordnet sind. Wenn mehrere Magnetelemente vorgesehen sind, können diese sowohl an dem Funktionsbauteil als auch an dem feststehenden Bauteil angeordnet sein. Bei dem feststehenden Bauteil handelt es sich beispielsweise um einen Gehäuseabschnitt des Antriebsmoduls oder ein Bauteil, welches am Gehäuse fest oder lösbar montiert ist, welches auch einstellbar ausgeführt sein kann. Auch das Anbringen eines oder mehrerer Magnetelemente an der Antriebseinrichtung, die bevorzugt als ein Elektromotor ausgeführt ist, kann vorgesehen sein.

Eine vorteilhafte Ausführungsform der Erfindung sieht vor, dass das eine oder die mehreren Magnetelemente als Element nach mindestens einer Bauart ausgewählt aus der folgenden Gruppe von Bauarten gebildet sind: Permanentmagnetelement, Elektromagnetelement, abschaltbares Magnetelement und hinsichtlich einer aufgebrachten Magnetkraft regelbares Magnetelement. Permanentmagnetelemente haben den Vorteil, dass sie ein Magnetfeld zur Verfügung stellen, ohne das es weiterer Hilfsmittel bedarf. Ein Vorteil von Elektromagnetelementen besteht darin, dass diese aufgabenabhängig mit einem Strom beaufschlagt werden können. Hierzu ist es notwendig, dass an das beispielsweise als eine Spule ausgeführte Elektromagnetelement eine Spannung angelegt wird. In dem Antriebsmodul kann die hierfür notwendige Spannung beispielsweise aus der elektrischen Spannungsversorgung für die Antriebseinrichtung entnommen werden. Mittels Einstellen einer Stromstärke sind Elektromagnetelemente auch regelbar.

Bevorzugt sieht eine Fortbildung der Erfindung vor, dass die mehreren Magnetelemente gegenseitig zugeordnet Magnetelemente umfassen, die wahlweise auf Lücke versetzt angeordnet sind. Eine zweckmäßige Ausgestaltung sieht beispielsweise vor, dass einander gegenüberliegende Magnetelemente auf Lücke versetzt angeordnet sind. Aber auch ein direktes einander Gegenüberliegen kann vorgesehen sein. Hierbei kann die Anordnung der zugeordneten Magnetelemente so erfolgen, dass sich einander zugeordnete Magnetelemente gegenseitig anziehen, wodurch ein Verdrehen des Funktionsbauteils verhindert wird. Alternativ oder ergänzend kann auch vorgesehen sein, dass einander zugeordnete Magnetelemente sich abstoßen und hierdurch ein Verdrehen des Funktionsbauteils unterbunden wird.

Bei einer vorteilhaften Ausgestaltung der Erfindung kann vorgesehen sein, dass mit wenigstens einem an dem Funktionsbauteil gebildeten Magnetelement an einer Kopplungsaufnahme an dem Funktionsbauteil eine magnetische Kopplungskraft zum lösbaren Ankoppeln eines Kopplungsbauteils oder einer Nadel an die Kopplungsaufnahme bereitgestellt ist. Das wenigstens eine an dem Funktionsbauteil gebildete Magnetelement kann an dem Funktionsbauteil so angeordnet sein, dass einerseits rückseitig eine magnetische Rückhaltekraft entwickelt wird, um dem Verdrehen des Funktionsbauteils entgegen zu wirken, und andererseits in Richtung der Vorderseite des Funktionsbauteils eine weitere Magnetkraft zur Verfügung gestellt ist, über die das Kopplungsbauteil oder eine Nadel an eine Kopplungsaufnahme an dem Funktionsbauteil gekoppelt werden. Die zur Ankopplung zur Verfügung gestellte Magnetkraft kann ergänzend zu einer mechanischen Kopplung der Nadel oder des Kopplungsbauteils in der Kopplungsaufnahme wirken. Aber auch ein alleiniges Ankoppeln mit Hilfe der magnetischen Kopplungskraft kann vorgesehen sein. Zum Beispiel kann ein an dem Kopplungsbauteil oder an der Nadel gebildeter Kopf in einer als Vertiefung ausgeführte Kopplungsaufnahme eingreifen und hierin mittels der magnetischen Kopplungskraft gehalten werden. Die magnetische Kopplungskraft kann in diesem Zusammenhang so ausgelegt werden, dass das Kopplungsbauteil oder die Nadel auch nach der Aufnahme schwenkbar sind.

Eine Weiterbildung der Erfindung kann vorsehen, dass das Funktionsbauteil mittels eines Kugellagers freilaufend gelagert ist.

Bei einer vorteilhaften Ausgestaltung der Erfindung kann eine mechanische Verdrehsicherung für das Funktionsbauteil vorgesehen sein. Die magnetische und die mechanische Verdrehsicherung ergänzen sich in dieser Ausgestaltung. In einer Ausführung kann vorgesehen sein, dass die mechanische Verdrehsicherung nur Wirkung entfaltet, wenn die dem Verdrehen des Funktionsbauteils entgegengesetzte Magnetkraft zuvor überwunden wurde, was also ohne die zusätzliche Sicherung mit der mechanischen Verdrehsicherung zum Verdrehen des Funktionsbauteils führen würde. Die mechanische Verdrehsicherung dient dann als eine Art Anschlag nach dem "Durchbrechen" der Magnetkraft. Eine solche Situation kann insbesondere am Beginn des Betriebs der Vorrichtung auftreten oder bei einer Störung. Die dem Verdrehen des Funktionsbauteils entgegenwirkende Magnetkraft ist in einer zweckmäßigen Ausgestaltung so gewählt, dass es im Normalbetrieb ansonsten bei einer kontaktlosen oder kontaktfreien Verdrehsicherung bleibt, die mittels der magnetischen Verdrehsicherung gebildet ist. Hierzu können die verwendeten Magnetelemente, die in verschiedenen Größen verfügbar sind, entsprechend ausgelegt werden.

Bevorzugt sieht eine Fortbildung der Erfindung vor, dass die magnetische Verdrehsicherung eine zum Eingriff der mechanischen Verdrehsicherung führende Bewegung dämpfend gebildet ist, wodurch insbesondere die Geräuschvermeidung weiter unterstützt wird. Zu Einstellen des Dämpfverhaltens können die verwendeten Magnetelemente entsprechend ihrer zur Verfügung gestellten Magnetkraft ausgewählt werden.

Eine Weiterbildung der Erfindung kann vorsehen, dass die mechanischen Verdrehsicherung rückseitige Vorsprünge an dem Funktionsbauteil sowie zugeordnete Gegenvorsprünge aufweist die im Eingriff stehen.

Eine vorteilhafte Ausführungsform der Erfindung sieht vor, dass Kopplungsbauteil mit einem Neigungswinkel von etwa 7° bis etwa 8° zu einer Antriebswelle angeordnet ist, auf welcher das Funktionsbauteil freilaufend gelagert ist.

Im folgenden werden vorteilhafte Ausgestaltungen des Handgerätes zum lokalen Aufstechen einer menschlichen oder einer tierischen Haut näher erläutert.

Eine bevorzugte Weiterbildung der Erfindung sieht vor, dass die Nadeleinrichtung direkt an eine Kopplungsaufnahme an dem Funktionsbauteil magnetisch gekoppelt ist.

Bei einer zweckmäßigen Ausgestaltung der Erfindung kann vorgesehen sein, dass die Nadeleinrichtung über einen Kopplungsmechanismus an eine Kopplungsaufnahme an dem Funktionsbauteil magnetisch gekoppelt ist. Hierbei kann eine magnetische Kopplung zwischen der Nadeleinrichtung, insbesondere auch direkt mit der Nadel, und dem Kopplungsmechanismus und / oder zwischen dem Kopplungsmechanismus und der Kopplungsaufnahme an dem Funktionsbauteil vorgesehen sein. Als Kopplungsmechanismus dient zum Beispiel ein Pleuel, welches endseitig mit einem oder zwei Kugelköpfen versehen sein kann.

Eine vorteilhafte Ausführungsform der Erfindung sieht vor, dass ein von dem Kopplungsmechanismus umfasstes Kopplungsbauteil, welches wahlweise direkt an die Kopplungsaufnahme gekoppelt ist, einen Kopplungspunkt zur Übernahme der zur Verfügung gestellten Vorwärts- / Rückwärtsbewegung seitlich versetzend, wahlweise zur einer Mitteachse hin, gebildet ist. Das Kopplungsbauteil ist bevorzugt als ein Pleuel ausgeführt, bei dem in einer zweckmäßigen Ausgestaltung endseitig Kugelköpfe gebildet sind, die beim Ankoppeln in zugeordnete Aufnahmen greifen.

Bevorzugt sieht eine Fortbildung der Erfindung vor, dass das Antriebsmodul und das Bearbeitungsmodul lösbar verbunden sind.

Bei einer vorteilhaften Ausgestaltung der Erfindung kann vorgesehen sein, dass das Bearbeitungsmodul ein Einwegmodul ist.

Eine Weiterbildung der Erfindung kann vorsehen, dass das Bearbeitungsmodul, wahlweise mittels Hinzufügen eines oder mehrer Ergänzungsbauteile, einer Modulbauart aus gewählt aus der folgenden Gruppe von Modulbauarten entsprechend konfiguriert ist: Tattoo-Modul, Permanent-Make-up-Modul und Wirkstoffabgabemodul.

### Beschreibung bevorzugter Ausführungsformen der Erfindung

Die Erfindung wird im folgenden anhand von bevorzugten Ausführungsformen unter Bezugnahme auf Figuren einer Zeichnung näher erläutert. Hierbei zeigen:
- Fig. 1: eine Darstellung einer Ausführungsform einer Vorrichtung zum lokalen Aufstechen einer menschlichen oder einer tierischen Haut,
- Fig. 2: eine vergrößerte Darstellung eines Abschnitts der Vorrichtung aus Fig. 1 und
- Fig. 3: eine perspektivische Darstellung einer Vorrichtung zum lokalen Aufstechen einer menschlichen oder einer tierischen Haut in einer weiteren Ausführung.

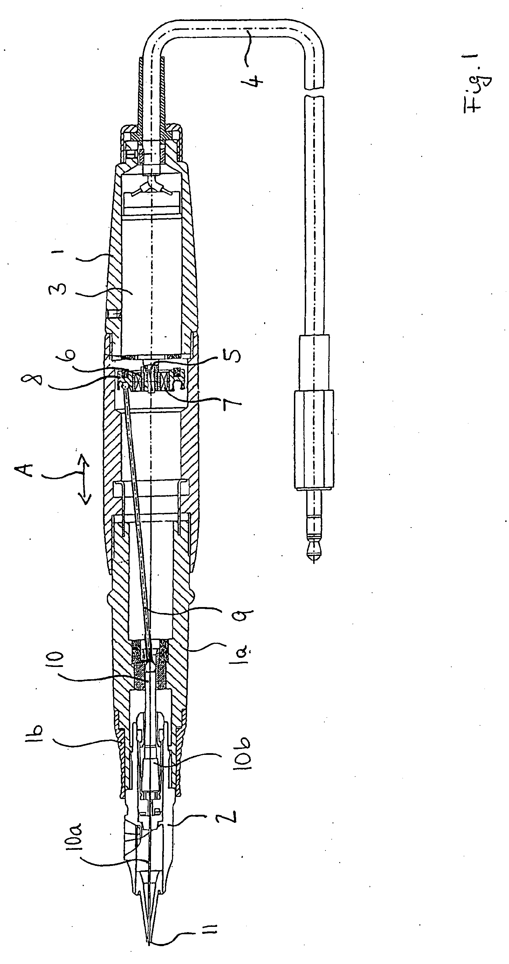

Fig. 1 zeigt eine Darstellung einer Vorrichtung zum lokalen Aufstechen einer menschlichen oder einer tierischen Haut mit einem Antriebsmodul 1 und einem hieran lösbar befestigten Bearbeitungsmodul 2. Die lösbare Verbindung ist bevorzugt mittels einer Schraub- oder einer Steckverbindung gebildet. Das Antriebsmodul 1 weist einen oberen Gehäuseabschnitt 1a und ein sich hieran anschließendes Griffstück 1b auf, welches in einer anderen Ausführungsform (nicht dargestellt) an dem Bearbeitungsmodul 2 gebildet sein kann. In das Antriebsmodul 1 ist ein Motor 3 integriert, welcher über eine Versorgungsleitung 4 an eine Spannungsversorgung angeschlossen werden kann, so dass mit dem Motor 3 eine Antriebsdrehbewegung einer Antriebswelle 5 erzeugt werden kann.

Auf der Antriebswelle 5 ist feststehend eine Taumelbuchse 6 gelagert, auf die ein Kugellager 7 mit einem Innenring und einem Außenring aufgesetzt ist. An das Kugellager 7 ist ein Funktionsbauteil 8 freilaufend gekoppelt, welches in Folge der Antriebsdrehbewegung der Antriebswelle 5 eine Taumel- oder Kippbewegung ausführt, so dass auf ein als Pleuel ausgeführtes Kopplungsbauteil 9 eine Antriebskraft für eine Vorwärts- und Rückwärtsbewegung eingeleitet wird, die in Fig. 1 mittels eines Pfeils A schematisch dargestellt ist. Über das Kopplungsbauteil 9 wird die Antriebskraft für die Vorwärts- und Rückwärtsbewegung auf eine in einer Nadeleinrichtung 10 aufgenommene Nadel 10a gegeben. Hierdurch wird die in einer Nadelaufnahme 10b gehaltene Nadel 10a, bei der es sich um eine Einzelnadel oder ein Nadelsystem handeln kann, durch eine Nadeldüse 11 hindurch repetierend aus- und eingefahren.

Es kann auch vorgesehen sein, eine Antriebskraft nur für die Vorwärtsbewegung zur Verfügung zu stellen. Die Rückholung der Nadeleinrichtung 10b erfolgt dann mit anderen Mitteln, zum Beispiel mittels einer an die Nadeleinrichtung 10b gekoppelten Federmembran, zum Beispiel aus einem elastischen Material wie Gummi oder Kunststoff. Auch eine Rückholfeder kann alternativ oder ergänzend vorgesehen sein.

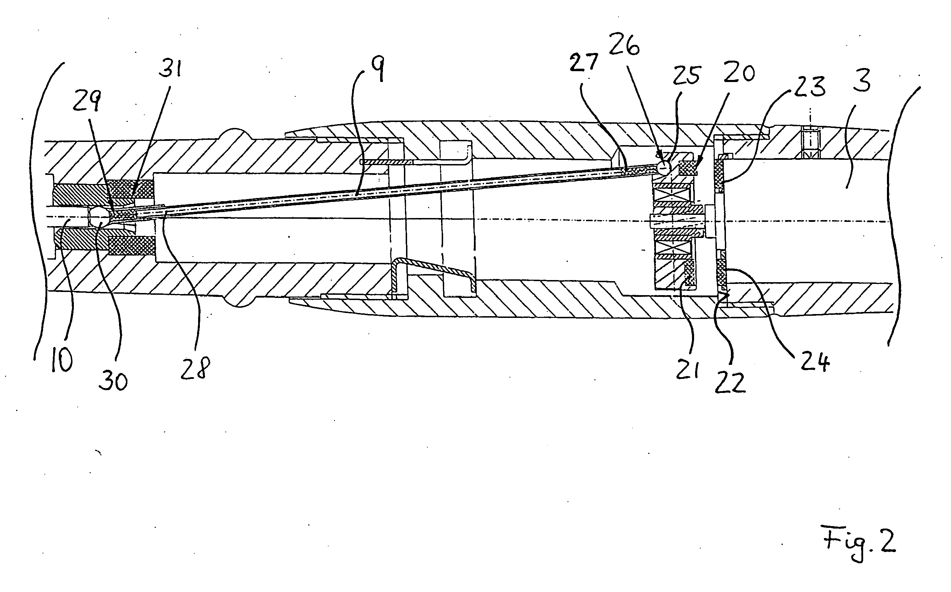

Die Taumelbuchse 6, das Kugellager 7 sowie das hiermit gekoppelte Funktionsbauteil 8, welches mit einem unten unter Bezugnahme auf Fig. 2 näher erläuterten Verdrehsicherung gegen ein Verdrehen gesichert ist, und das Kopplungsbauteil 9 sind Bestandteil eines Wandlungsmechanismus, mit dem die Antriebsdrehbewegung der Antriebswelle 5 in eine in die Nadeleinrichtung 10 einzukoppelnde, lineare Bewegung gewandelt wird. Zum Wandlungsmechanismus gehören hierbei auch zumindest Teilelemente der Verdrehsicherung, die unten näher beschrieben wird.

Fig. 2 zeigt eine vergrößerte Darstellung eines Abschnitts der Vorrichtung aus Fig. 1. Für gleiche Merkmale werden in Fig. 2 wenigstens teilweise die Bezugszeichen wie in Fig. 1 verwendet.

Das Funktionsbauteil 8 weist gemäß Fig. 2 Magnetelemente 20, 21 auf, die rückseitig in zugeordnete Vertiefungen eingesetzt sind. Auf einer dem Funktionsbauteil 8 gegenüberliegenden Fläche 22 sind den Magnetelementen 20, 21 zugeordnete Magnetelemente 23, 24 an dem Motor 3 gebildet. Mit Hilfe der Magnetelemente 20, 21 an dem Funktionsbauteil 8 sowie der zugeordneten Magnetelemente 23, 24 an dem Motor 3 ist eine Verdrehsicherung für das freilaufende Funktionsbauteil 8 gebildet. Die verschiedenen Magnetelemente können hinsichtlich ihrer Anordnung und ihrer magnetischen Ausgestaltung so gewählt werden, dass sich einander zugeordnete Magnetelemente entweder anziehen oder abstoßen. Mit Hilfe beider Ausgestaltungen wird eine magnetische Rückhaltekraft zur Verfügung gestellt, die ein Verdrehen des Funktionsbauteils 8 nahezu oder vollständig unterbindet. Die Magnetelementen 20, 21 und die zugeordneten Magnetelemente 23, 24 sind bei einer zweckmäßigen Ausführung in einer Ausgangsstellung von Funktionsbauteil 8 und Motor 3 relativ zueinander versetzt angeordnet. Beispielsweise können entlang einer Umfangslinie abwechselnd eines der Magnetelemente 20, 21 und eines der zugeordneten Magnetelemente 23, 24 gebildet sein, die bevorzugt auf Lücke versetzt sind.

Vorderseitig ist an dem Funktionsbauteil 8 eine Kopplungsaufnahme 25 als eine Vertiefung gebildet, in welcher ein kugelförmiger Kopf 26 des bevorzugt als Pleuel ausgeführten Kopplungsbauteils 9 gelagert ist, welches insbesondere der Einkopplung der Antriebskraft auf die Nadeleinrichtung 10 dient. Benachbart zu dem Kopf 26 ist an dem Kopplungsbauteil 9 ein weiteres Magnetelement 27 angeordnet, welches zusammen mit dem rückseitig angeordneten Magnetelement 20 an dem Funktionsbauteil 8 eine magnetische Haltekraft zur Verfügung stellt, mit der der Kopf 26 in der Kopplungsaufnahme 25 gehalten wird.

Gemäß Fig. 2 weist das Kopplungsbauteils 9 an einem der Nadeleinrichtung 10 zugewandten Endabschnitt 28 ein anderes Magnetelement 29 auf, welches in dem Endabschnitt 28 einer Kugel 30 nachgelagert ist. Das andere Magnetelement 29 wirkt mit einem zusätzlichen Magnetelement 31 zusammen, welches den Endabschnitt 28 des Kopplungsbauteils 9 zumindest in Teilbereichen vollständig oder teilweise umgreift und ein- oder mehrteilig ausgeführt sein kann. Hierdurch wird eine magnetische Zusatzkraft bereitgestellt, die einem Herausbewegen des Endabschnittes 28 aus dem Bereich des zusätzlichen Magnetelementes 31 entgegenwirkt und so insbesondere eine Rückstellkraft zur Verfügung stellt. Alternativ oder ergänzend kann vorgesehen sein, die Kugel 30 aus einem magnetischen Material zu bilden oder in die Kugel 30 ein Magnetelement zu integrieren.

Soweit für die verschiedenen Bauelemente oder Teilabschnitte hiervon ein Material verwendet wird, welches magnetisch mit einem der beschriebenen Magnetelemente aufgrund seiner natürlichen Eigenschaften in Wechselwirkung tritt, kann die jeweilige Magnetkraftkopplung auch auf Basis dieser Wechselwirkung gebildet sein.

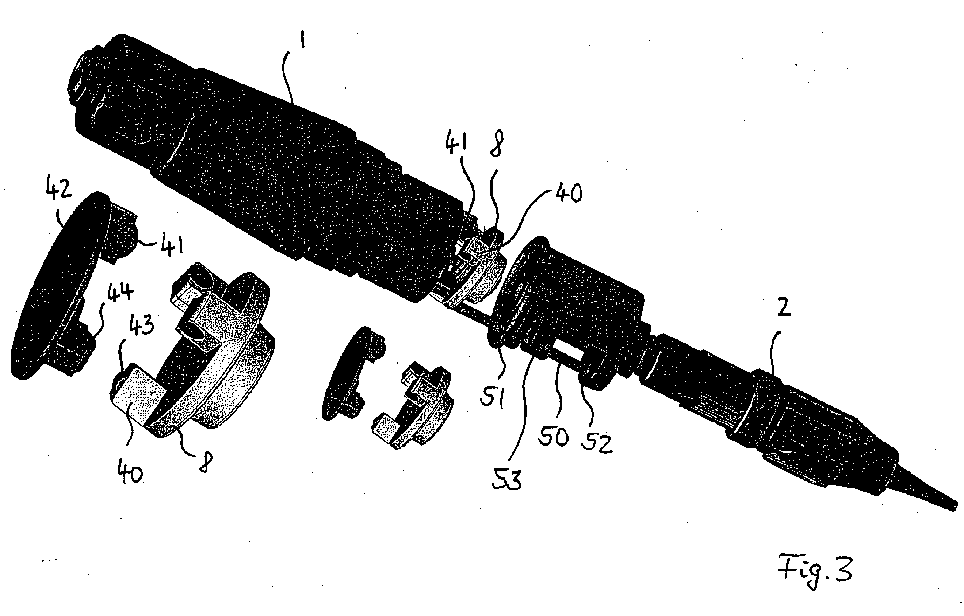

Fig. 3 zeigt eine perspektivische Darstellung einer Vorrichtung zum lokalen Aufstechen einer menschlichen oder einer tierischen Haut in einer weiteren Ausführung. In Fig. 3 werden für gleiche Merkmale die selben Bezugszeichen wie in den Fig. 1 und 2 verwendet.

An dem Antriebsmodul 1 ist das Bearbeitungsmodul 2 lösbar befestigt. Die lösbare Verbindung ist bevorzugt mittels einer Schraub- oder einer Steckverbindung gebildet.

Im Unterschied zu der Ausführungsform in den Fig. 1 und 2 ist das Funktionsbauteil 8 bei der Ausgestaltung nach Fig. 3 zunächst mittels einer mechanischen Verdrehsicherung gegen ein Verdrehen beim Ausführen der Taumel- oder Kippbewegung gesichert. Hierzu sind an dem Funktionsbauteil 8 rückseitige Vorsprünge 40 gebildet, die bei der dargestellten Ausführung umlaufend gleichmäßig beabstandet angeordnet sind. Die rückseitigen Vorsprünge 40 greifen mit zugeordneten Gegenvorsprüngen 41 ineinander, die ihrerseits an einer Scheibe 42 gebildet sind, ebenfalls umlaufend gleichmäßig beabstandet. Mittels Fixieren der Scheibe 42, wodurch die Gegenvorsprünge 41 feststehend sind, ist so das auf der Antriebswelle 5 freilaufende Funktionsbauteil 8 beim Betrieb gegen ein Verdrehen auch mechanisch gesichert. Im eingebauten Zustand ist ein Abstand zwischen der Rückseite des Funktionsbauteils 8 und der Vorderseite der Scheibe 42 so eingestellt, dass das Funktionsbauteil 8 die Taumel- oder Kippbewegung ausführen kann.

Alternativ oder ergänzend zu der vorangehend beschriebenen mechanischen Verdrehsicherung des Funktionsbauteils 8, bei der die Verdrehsicherung direkt an dem Funktionsbauteil 8 gebildet ist, kann eine Verdrehsicherung in mechanischer Ausbildung auch auf andere Art und Weise, nämlich nicht direkt an dem Funktionsbauteil 8 gebildet werden. So kann in einer Ausgestaltung (nicht dargestellt) vorgesehen sein, dass mit einem Haltebauteil (vgl. Bezugszeichen 53 unten) eine Verdrehsicherung erreicht ist. In diesem Fall wird der Anpressdruck einer hiermit zusammenwirkenden Feder geeignet gewählt und / oder eine Mulde für das Ende des Kopplungsbauteils 9 auf dem Funktionsbauteil 8 vorgesehen. Die mechanische Verdrehsicherung kann beispielsweise in dem Bearbeitungsmodul 2 gebildet sein. Dieses wird in einer Ausgestaltung beispielsweise dadurch realisiert, dass ein Pleuel, Durchbrüche und ein Haltebauteil (vgl. Bezugszeichen 50, 51, 52 und 53 unten) sowie das Kopplungsbauteil 9 und entsprechende Gehäuseteile in das Bearbeitungsmodul 2 verlagert sind.

Zusätzlich zu der mechanischen Verdrehsicherung ist bei der Ausführungsform nach Fig. 3 mit Hilfe von erstem Magnetelementen 43 an dem Funktionsbauteil 8 und diesen zugeordneten zweiten Magnetelementen 44 an der Scheibe 42 eine magnetische Kopplung zwischen dem Funktionsbauteil 8 und der Scheibe 42 gebildet, die ebenfalls die Funktionalität einer Verdrehsicherung implementiert. Bei geeigneter Materialauswahl kann die magnetische Kopplung auch erreicht werden, indem entweder nur an dem Funktionsbauteil 8 oder nur an der Scheibe 42 Magnetelemente gebildet sind, die dann mit zugeordneten Materialbereichen an dem jeweils anderen Bauteil magnetisch wechselwirken.

Mechanische uns magnetische Verdrehsicherung wirken beide einem Verdrehen des Funktionsbauteils 8 entgegen, wobei die magnetische Kopplung zwischen dem Funktionsbauteil 8 und der Scheibe 42 dämpfend wirkt, wenn die rückseitigen Vorsprünge 40 und die Gegenvorsprünge 41 beim Betrieb seitlich zur Anlage kommen. Beim Starten des Betriebs der Vorrichtung zum lokalen Aufstechen einer menschlichen oder einer tierischen Haut wirkt zunächst die abstoßende Magnetkraft zwischen den Magnetelementen 43, 44 als Rückhaltekraft dem Verdrehen des Funktionsbauteils 8 entgegen, bevor es dann bedarfsweise zum Kontakt zwischen den rückseitigen Vorsprüngen 40 und den Gegenvorsprüngen 41 kommt.

Gemäß Fig. 3 liegt auf der Vorderseite des Funktionsbauteils 8 ein rückseitiges Ende eines Pleuels 50 auf, welches seinerseits in Längsrichtung der Vorrichtung zum lokalen Aufstechen verschiebbar in zwei Durchbrüchen 51, 52 gelagert ist. Auf dem Pleuel 50 ist festsitzend ein Haltebauteil 53 angeordnet, welches gegen eine mittels einer Feder ausgebildete Vorspannung in Richtung des Bearbeitungsmoduls 2 gedrückt werden kann, indem das Funktionsbauteil 8 seine Taumel- oder Kippbewegung ausführt. Im Laufe dieser Taumel- und Kippbewegung des Funktionsbauteils 8 wird das Haltebauteil 53 dann auch zusammen mit dem Pleuel 50 aufgrund der Feder (nicht dargestellt) wieder zurück gedrückt in die in Fig. 3 dargestellte Stellung. Mit Hilfe dieser Vor- und Rückbewegung von Pleuel 50 und Haltebauteil 53 wird ein Aus- und Einfahren der Nadelspitze zum wiederholten Aufstechen der Haut erreicht. Das Pleuel 50 ist in den beiden Durchbrüchen 51, 52 in hierin eingesetzten Lagern angeordnet, die ein leichtgängiges Gleiten des Pleuels 50 bei der Vor- und Rückbewegung unterstützen. Das rückseitige Ende des Pleuels 50 lagert auf der im wesentlichen eben ausgebildeten Fläche auf der Vorderseite des Funktionsbauteils 8.

Unabhängig von den verschiedenen Ausführungsformen ist vorgesehen, dass das Kopplungsbauteil 9 mit einem Neigungswinkel von etwa 7° bis etwa 8° zu der Antriebswelle 5 angeordnet ist, auf welcher das Funktionsbauteil 8 freilaufend gelagert ist.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Antriebsmodul für eine Vorrichtung zum lokalen Aufstechen einer menschlichen oder einer tierischen Haut, mit einer Antriebseinrichtung (3), die konfiguriert ist, eine Antriebsdrehbewegung zu erzeugen, und einem an die Antriebseinrichtung (3) gekoppelten Wandlungsmechanismus, der konfiguriert ist, die Antriebsbewegung in eine auf eine die Haut lokal aufstechende Nadeleinrichtung (10) einkoppelbare Vorwärts- / Rückwärtsbewegung zu wandeln, und der ein freilaufendes Funktionsbauteil (8) umfasst, **dadurch gekennzeichnet, dass** das Funktionsbauteil (8) an eine eine Taumel- oder Kippbewegung des Funktionsbauteils (8) zulassende, magnetische Verdrehsicherung gekoppelt ist, mit der eine einem Verdrehen des Funktionsbauteils (8) entgegenwirkende, magnetische Rückhaltekraft zur Verfügung gestellt ist.

2. Antriebsmodul nach Anspruch 1, **dadurch gekennzeichnet, dass** die magnetische Verdrehsicherung als berührungslose Verdrehsicherung gebildet ist, die konfiguriert ist, die magnetische Rückhaltekraft berührungslos auf das Funktionsbauteil (8) einzukoppeln.

3. Antriebsmodul nach Anspruch 1 oder 2, **dadurch gekennzeichnet, dass** die magnetische Verdrehsicherung mit einem oder mehreren Magnetelementen (20, 21, 23, 24; 43, 44) gebildet ist, die wahlweise an dem Funktionsbauteil (8) und / oder an einem feststehenden Bauteil angeordnet sind.

4. Antriebsmodul nach Anspruch 3, **dadurch gekennzeichnet, dass** das eine oder die mehreren Magnetelemente (20, 21, 23, 24; 43, 44) als Element nach mindestens einer Bauart ausgewählt aus der folgenden Gruppe von Bauarten gebildet sind: Permanentmagnetelement, Elektromagnetelement, abschaltbares Magnetelement und hinsichtlich einer aufgebrachten Magnetkraft regelbares Magnetelement.

5. Antriebsmodul nach Anspruch 3 oder 4, **dadurch gekennzeichnet, dass** die mehreren Magnetelemente (20, 21, 23, 24; 43, 44) gegenseitig zugeordnete Magnetelemente umfassen, die wahlweise auf Lücke versetzt angeordnet sind.

6. Antriebsmodul nach einem der Ansprüche 3 bis 5, **dadurch gekennzeichnet, dass** mit wenigstens einem an dem Funktionsbauteil (8) gebildeten Magnetelement an einer Kopplungsaufnahme (25) an dem Funktionsbauteil (8) eine magnetische Kopplungskraft zum lösbaren Ankoppeln eines Kopplungsbauteils (9) oder einer Nadeleinrichtung (10) an die Kopplungsaufnahme (25) bereitgestellt ist.

7. Antriebsmodul nach einem der vorangehenden Ansprüche, **dadurch gekennzeichnet, dass** das Funktionsbauteil (8) mittels eines Kugellagers (7) freilaufend gelagert ist.

8. Antriebsmodul nach einem der vorangehenden Ansprüche, **gekennzeichnet durch** eine mechanische Verdrehsicherung (40, 41) für das Funktionsbauteil (8).

9. Antriebsmodul nach Anspruch 8, **dadurch gekennzeichnet, dass** die magnetische Verdrehsicherung eine zum Eingriff der mechanischen Verdrehsicherung (40, 41) führende Bewegung dämpfend gebildet ist.

10. Antriebsmodul nach Anspruch 8 oder 9, **dadurch gekennzeichnet, dass** die mechanischen Verdrehsicherung (40, 41) rückseitige Vorsprünge (40) an dem Funktionsbauteil (8) sowie zugeordnete Gegenvorsprünge (41) aufweist die im Eingriff stehen.

11. Antriebsmodul nach einem der Ansprüche 6 bis 10, **dadurch gekennzeichnet, dass** Kopplungsbauteil (9) mit einem Neigungswinkel von etwa 7° bis etwa 8° zu einer Antriebswelle (5) angeordnet ist, auf welcher das Funktionsbauteil (8) freilaufend gelagert ist.

12. Handgerät zum lokalen Aufstechen einer menschlichen oder einer tierischen Haut, mit einem Antriebsmodul (1) nach mindestens einem der vorangehenden Ansprüche und einem Bearbeitungsmodul (2), in dem eine eine Nadel aufweisende Nadeleinrichtung (10) gebildet ist, welche konfiguriert ist, die von dem Antriebsmodul (1) zur Verfügung gestellte Vorwärts- / Rückwärtsbewegung in eine Stechbewegung umzusetzen.

13. Handgerät nach Anspruch 12, **dadurch gekennzeichnet, dass** die Nadeleinrichtung (10) direkt an eine Kopplungsaufnahme (25) an dem Funktionsbauteil (8) magnetisch gekoppelt ist.

14. Handgerät nach Anspruch 12, **dadurch gekennzeichnet, dass** die Nadeleinrichtung (10) über einen Kopplungsmechanismus (9) an eine Kopplungsaufnahme (25) an dem Funktionsbauteil magnetisch gekoppelt ist.

15. Handgerät nach Anspruch 14, **dadurch gekennzeichnet, dass** ein von dem Kopplungsmechanismus umfasstes Kopplungsbauteil (9), welches wahlweise direkt an die Kopplungsaufnahme (25) gekoppelt ist, einen Kopplungspunkt zur Übernahme der zur Verfügung gestellten Vorwärts- / Rückwärtsbewegung seitlich versetzend, wahlweise zur einer Mittelachse hin, gebildet ist.

16. Handgerät nach einem der Ansprüche 12 bis 15, **dadurch gekennzeichnet, dass** das Antriebsmodul (1) und das Bearbeitungsmodul (2) lösbar verbunden sind.

17. Handgerät nach Anspruch 16, **dadurch gekennzeichnet, dass** das Bearbeitungsmodul (2) ein Einwegmodul ist.

18. Handgerät nach einem der Ansprüche 12 bis 17, **dadurch gekennzeichnet, dass** das Bearbeitungsmodul (2), wahlweise mittels Hinzufügen eines oder mehrerer Ergänzungsbauteile, einer Modulbauart ausgewählt aus der folgenden Gruppe von Modulbauarten entsprechend konfiguriert ist: Tattoo-Modul, Permanent-Make-up-Modul und Wirkstoffabgabemodul.

## Claims

1. A drive module for a device for the local piercing of a human or an animal skin, with a drive apparatus (3) which is configured for the purpose of producing a drive rotary movement and a conversion mechanism coupled to the drive apparatus (3) which is configured for the purpose of converting the drive movement into a coupling-connectable forward/backward movement for a needle apparatus (10) piercing the skin locally, and which comprises a free-running functional member (8), **characterised in that** the functional member (8) is coupled to a magnetic anti-turn locking element allowing a wobble or tilting movement of the functional member (8) where, with the said anti-turn locking element, a magnetic retaining force is provided which acts against a turning of the functional member (8).

2. The drive module according to Claim 1, **characterised in that** the magnetic anti-turn locking element is formed as a non-contact anti-turn locking element which is configured for the purpose of coupling the magnetic retaining force in a noncontacting manner onto the functional member (8).

3. The drive module according to Claim 1 or 2, **characterised in that** the magnetic anti-turn locking element is formed with one or several magnet elements (20, 21, 23, 24; 43, 44) which are selectively arranged on the functional member (8) and/or on a stationary member.

4. The drive module according to Claim 3, **characterised in that** the one or the several magnet elements (20, 21, 23, 24; 43, 44) are formed from at least one structural type selected from the following group of structural types: permanent magnet element, electromagnet element, switch-off magnet element and, with regard to a produced magnetic force, a controllable magnet element.

5. The drive module according to Claim 3 or 4, **characterised in that** the several magnet elements (20, 21, 23, 24; 43, 44) comprise oppositely arranged magnet elements which are selectively arranged offset to gap.

6. The drive module according to any one of the Claims 3 to 5, **characterised in that,** with at least one magnet element formed on the functional member (8) on a coupling receptacle (25) on the functional member (8), a magnetic coupling force is provided for the detachable coupling of a coupling member (9) or a needle apparatus (10) to the coupling receptacle (25).

7. The drive module according to any one of the preceding Claims, **characterised in that** the functional member (8) is mounted in a free-running manner by means of a ball bearing (7).

8. The drive module according to any one of the preceding Claims, **characterised by** a mechanical anti-turn locking element (40, 41) for the functional member (8).

9. The drive module according to Claim 8, **characterised in that** the magnetic anti-turn locking element is formed with a damping effect for the movement for the intermesh of the mechanical anti-turn locking element (40, 41).

10. The drive module according to Claim 8 or 9, **characterised in that** the mechanical anti-turn locking element (40, 41) has rear-side projections (40) on the functional member (8) as well as allocated counter-projections (41) which are intermeshing.

11. The drive module according to any one of the Claims 6 to 10, **characterised in that** the coupling member (9) is arranged with a gradient angle of approximately 7° to approximately 8° to a drive shaft (5), on which the functional member (8) is mounted in a free-running manner.

12. A handheld for the local piercing of a human or an animal skin, with a drive module (1) according to at least one of the preceding Claims and a processing module (2) in which a needle apparatus (10) having a needle is formed and which is configured for the purpose of converting the forward/backward movement provided by the drive module (1) into a piercing movement.

13. The handheld according to Claim 12, **characterised in that** the needle apparatus (10) is magnetically coupled directly to a coupling receptacle (25) on the functional member (8).

14. The handheld according to Claim 12, **characterised in that** the needle apparatus (10) is magnetically coupled to a coupling receptacle (25) on the functional member by way of a coupling mechanism (9).

15. The handheld according to Claim 14, **characterised in that** a coupling member (9) encompassed by the coupling mechanism is formed, where said coupling member (9) is selectively coupled directly to the coupling receptacle (25), laterally offsetting a coupling point for the take-over of the available forward/backward movement, selectively towards a middle axis.

16. The handheld according to any one of the Claims 12 to 15, **characterised in that** the drive module (1) and the processing module (2) are detachably connected.

17. The handheld according to Claim 16, **characterised in that** the processing module (2) is a disposable module.

18. The handheld according to any one of the Claims 12 to 17, **characterised in that** the processing module (2), selectively by means of the addition of one or several supplementary members, is a module type selected from the following group of module types and is correspondingly configured: tattoo module, permanent make-up module and active ingredient discharging module.

## Revendications

1. Module d'entraînement pour un dispositif destiné au perçage local d'une peau humaine ou d'une peau animale, avec un équipement d'entraînement (3) qui est configuré pour générer un mouvement rotatif d'entraînement, et un mécanisme de conversion couplé à l'équipement d'entraînement (3), mécanisme de conversion configuré de manière à convertir le mouvement d'entraînement en un mouvement d'avance et de recul dans un équipement à aiguille (10) qui perce la peau localement et qui comprend un composant fonctionnel (8) libre, **caractérisé en ce que** le composant fonctionnel est couplé a un dispositif magnétique de protection contre la torsion qui autorise un mouvement de culbutement ou de basculement du composant fonctionnel (8), dispositif de protection contre la torsion qui met à disposition une force de retenue magnétique s'opposant à la torsion du composant fonctionnel (8).

2. Module d'entraînement selon la revendication 1, **caractérisé en ce que** le dispositif magnétique de protection contre la torsion est formé comme protection contre la torsion sans contact configurée de manière à appliquer la force de retenue magnétique sans contact sur le composant fonctionnel (8).

3. Module d'entraînement selon la revendication 1 ou 2, **caractérisé en ce que** le dispositif magnétique de protection contre la torsion est formé avec un ou plusieurs éléments magnétiques (20, 21, 23, 24 ; 43, 44) qui sont disposés en option sur le composant fonctionnel (8) et/ou sur un composant fixe.

4. Module d'entraînement selon la revendication 3, **caractérisé en ce que** le ou les éléments magnétiques (20, 21, 23, 24 ; 43, 44) sont formés comme éléments d'au moins un type sélectionné dans le groupe suivant de types: élément d'aimant permanent, élément d'électroaimant, élément magnétique déconnectable et élément magnétique réglable pour ce qui est d'une force magnétique appliquée.

5. Module d'entraînement selon la revendication 3 ou 4, **caractérisé en ce que** les éléments magnétiques (20, 21, 23, 24 ; 43, 44) comprennent des éléments magnétiques réciproquement affectés l'un à l'autre qui sont disposés en option en quinconce.

6. Module d'entraînement selon l'une quelconque des revendications 3 à 5, **caractérisé en ce qu'**avec au moins un élément magnétique formé sur le composant fonctionnel (8), une force de couplage magnétique est appliquée à un logement de couplage (25) sur le composant fonctionnel (8) pour le couplage débrayable d'un composant de couplage (9) ou d'un équipement à aiguille (10) au logement de couplage (25).

7. Module d'entraînement selon l'une quelconque des revendications précédentes, **caractérisé en ce que** le composant fonctionnel (8) est logé librement au moyen d'un roulement à billes (7).

8. Module d'entraînement selon l'une quelconque des revendications précédentes, **caractérisé par** une protection mécanique contre la torsion (40, 41) pour le composant fonctionnel (8).

9. Module d'entraînement selon la revendication 8, **caractérisé en ce que** la protection magnétique contre la torsion est formée de manière à atténuer un mouvement entraînant l'intervention de la protection mécanique contre la torsion (40, 41).

10. Module d'entraînement selon la revendication 8 ou 9, **caractérisé en ce que** la protection mécanique contre la torsion (40, 41) présente des saillies (40) arrières sur le composant fonctionnel (8) ainsi que des contre-saillies (41) en prise qui leur sont affectées.

11. Module d'entraînement selon l'une quelconque des revendications 6 à 10, **caractérisé en ce que** le composant de couplage (9) est disposé avec un angle d'inclinaison d'environ 7° à environ 8° par rapport à un arbre d'entraînement (5) sur lequel le composant fonctionnel (8) est logé librement.

12. Appareil à main pour le perçage local d'une peau humaine ou d'une peau animale, avec un module d'entraînement (1) selon au l'une des revendications précédentes et un module de traitement (2) dans lequel est formé un équipement à aiguille (10) présente une aiguille, équipement configuré de manière à convertir le mouvement d'avance et de recul mis à disposition par le module d'entraînement (1) en un mouvement de perçage.

13. Appareil à main selon la revendication 12, **caractérisé en ce que** l'équipement à aiguille (10 est directement couplé magnétiquement à un logement de couplage (25) sur le composant fonctionnel (8).

14. Appareil à main selon la revendication 12, **caractérisé en ce que** l'équipement à aiguille (10 est couplé magnétiquement par l'intermédiaire d'un mécanisme de couplage (9) à un logement de couplage (25) sur le composant fonctionnel.

15. Appareil à main selon la revendication 14, **caractérisé en ce qu'**est formé un mécanisme de couplage comprenant le composant fonctionnel (9) qui est en option directement couplé au logement de couplage (25), décalant un point de couplage latéralement pour la reprise du mouvement d'avance et de recul mis à disposition, en option vers un axe central.

16. Appareil à main selon l'une quelconque des revendications 12 à 15, **caractérisé en ce que** le module d'entraînement (1) et le module de traitement (2) sont reliés de manière amovible.

17. Appareil à main selon la revendication 16, **caractérisé en ce que** le module de traitement (2) est un module à usage unique.

18. Appareil à main selon l'une quelconque des revendications 12 à 17, **caractérisé en ce que** le module de traitement (2) est configuré, en option par ajout d'où ou de plusieurs composants complémentaires, conformément à un type de module sélectionné dans le groupe suivant de types de modules: module de tatouage, module de fond de teint permanent et module de décharge de matière active.
